(19) Europäisches Patentamt
European Patent Office
Office européen des brevets

(11) Publication number: 0 253 592 B1

(12) # EUROPEAN PATENT SPECIFICATION

(45) Date of publication of the patent specification: 30.05.90

(51) Int. Cl.⁵: C07C 217/60, C07C 233/22

(21) Application number: 87306131.1

(22) Date of filing: 10.07.87

(54) **A method for epimerizing an optically active benzylalcohol derivative.**

(30) Priority: 11.07.86 JP 164262/86

(43) Date of publication of application: 20.01.88 Bulletin 88/3

(45) Publication of the grant of the patent: 30.05.90 Bulletin 90/22

(84) Designated Contracting States: AT BE CH DE ES GB GR IT LI LU NL SE

(56) References cited: AT-B- 338 242

(73) Proprietor: TANABE SEIYAKU CO., LTD., 2-10, Dosho-machi 3-chome, Chuo-ku Osaka(JP)

(72) Inventor: Gaino, Mitsunori, RC-5-306 Sunadanchi No. 4-1, Higashi-Omiya 3-chome, Omiya-shi Saitama-ken(JP)
Inventor: Iwakuma, Takeo, No. 5-7, Fujimi 1-chome, Ageo-shi Saitama-ken(JP)
Inventor: Komoto, Tadayuki, No. 2-43-702, Nankonaka 2-chome Suminoe-ku, Osaka-shi Osaka-fu(JP)
Inventor: Kawaguchi, Takayuki, No. 15-2-406, Sugamo 1-chome Toshima-ku, Tokyo-to(JP)
Inventor: Yamoto, Eisaku, No. 5-13, Suzurandai-Kitamachi 6-chome Kita-ku, Kobe-shi Hyogo-ken(JP)

(74) Representative: Hardisty, David Robert et al, BOULT, WADE & TENNANT 27 Furnival Street, London EC4A IPQ(GB)

EP 0 253 592 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

This invention relates to a method for epimerizing an optically active benzylalcohol derivative. More particularly, it relates to a method for epimerizing an optically active benzylalcohol derivative of the formula:

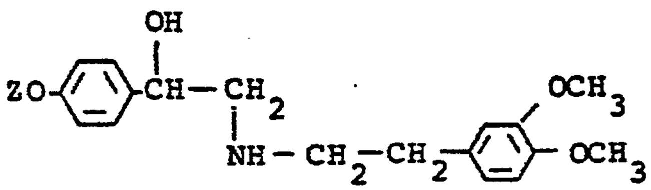

wherein ZO is a protected or unprotected hydroxy group.

It is known that ℓ-4-hydroxy-α-(3,4-dimethoxyphenethylaminomethyl)benzylalcohol is useful as a cardiotonic agent and also that said ℓ-isomer can be prepared by optical resolution of dℓ-4-benzyloxy-α-(3,4-dimethoxyphenethylaminomethyl)benzylalcohol, followed by debenzylation thereof (Japanese Patent Publication (examined) No. 16501/1980).

According to the above mentioned method, an undesired d-isomer (i.e., d-4-benzyloxy-α-(3,4-dimethoxyphenethylaminomethyl)benzylalcohol) is also prepared in the step of the optical reolution. If said undesired d-isomer can be epimerized by some method or other, all of the racemic modification supplied as the starting material can be converted into the desired ℓ-isomer by combination of the optical resolution and the epimerization. However, any method for epimerizing the undesired d-isomer has not been known up to now.

As a result of various investigations, we have now found a novel method which makes it possible to epimerize an optically active benzylalcohol derivative (I).

According to the present invention, an optically active benzylalcohol derivative (I) can be epimerized by the steps of:

(a) condensing said optically active benzylalcohol derivative (I) or a salt thereof with a carboxylic acid compound of the formula:
$R^1COOH$ (II)
wherein $R^1CO$ is an acyl group, or a reactive derivative thereof to give an optically active N-acylbenzylalcohol derivative of the formula :

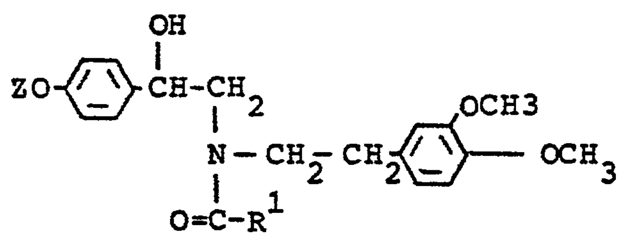

(III)

wherein $R^1CO$ and ZO are the same as defined above,
(b) subjecting the optically active N-acylbenzylalcohol derivative (III) to intramolecular cyclization in the presence of a thionyl halide, and
(c) treating the product obtained above with an alkali agent.

Although it is preferred to use an optically pure isomer of the benzylalcohol derivative (I) or a salt thereof as the starting material, an optically impure isomer thereof (i.e., a mixture of unequal amount of d- and ℓ-isomers) can also be used as the starting material.

In case where the group ZO in the compound (I) is a protected hydroxy group, examples of the protecting group include a substituted or unsubstituted benzyl group such as benzyl or p-methoxybenzyl; and a substituted or unsubstituted benzyloxycarbonyl gorup such as benzyloxycarbonyl or p-methoxybenzyloxycarbonyl.

Examples of the salt of the benzylalcohol derivative (I) include inorganic acid addition salts such as hydrochloride, hydrobromide or sulfate; and organic acid addition salts such as methanesulfonate, fumarate or maleate.

The condensation reation of the optically active benzylalcohol derivative (I) or a salt thereof with the carboxylic acid compound (II) or a reactive derivative thereof can be conducted in conventional manners. For example, the condensation reaction of the compound (I) or a salt thereof with the compound (II)

in its free form can be carried out in the presence of a condensing agent in a solvent. Examples of the carboxylic acid compound include those of the formula (II) in which $R^1CO$ is formyl group, a lower alkanoyl groups such as acetyl, propionyl or butyryl, an aralkylcarbonyl group such as benzylcarbonyl and an arylcarbonyl such as benzoyl, 4-nitrobenzoyl, 1-napthoyl or 2-napthoyl. Examples of the condensing agent include diphenyl phosphorazidate, diethyl phosphorazidate, diphenyl phosphorochloridate, diethyl phosphorocyanidate, boron trifluoride etherate, stannic chloride or 1-methyl-2-halopyridinium iodide. Dimethyl ether, tetrahydrofuran, dioxane, methylene chloride, chloroform, benzene, toluene, xylene, dimethylformamide, dimethylsulfoxide and the like are suitable as the solvent. It is preferred to carry out the reaction at a temperature of 0 to 120 °C, especially 20 to 100 °C.

On the other hand, the condensation reaction of the compound (I) or a salt thereof with a reactive derivative of the compound (II) can be carried out either in the presence or absence of an acid acceptor in a solvent. Examples of the reactive derivative of the compound (II) include the correspnding acid halides (e.g., acid chloride, acid bromide), anhydrides, mixed anhydrides (e.g., acetic formic anhydride), active esters (e.g., p-nitrophenyl ester, 2,4-dinitrophenyl ester, succinimide ester, phtalimide ester, benzotriazole ester, 2-pyrrolidon-1-yl ester), acid azides and acid amides (e.g., imidazole amide, 4-substituted-imidazole amide, triazole amide). Methylene chloride, ethyl acetate, teterahydrofuran, toluene, chloroform and the like are suitable as the solvent. Moreover, suitable examples of the acid acceptor include alkali metal hydroxides (e.g., potassium hydroxide, sodidum hydroxide), alkali metal carbonates or bicarbonates (e.g., sodium carbonate, sodium bicarbonate, potassium carbonate, potassium bicarbonate), trialkyl amines (e.g., trimethylamine, triethylamine), N,N-dialkylanilines (e.g., N,N-dimethylaniline, N,N-diethylaniline), pyridine and N-alkylmorpholines (e.g., N-methylmorpholine). It is preferred to carry out the reaction at a temperature of - 50 to 20 °C, especially 0 to 20 °C.

The intramolecular cyclization of the optically active N-acylbenzylalcohol derivative (III) can be conducted in the presence of a thionyl halide (e.g., thionyl chloride or thionyl bromide) in a solvent. Methylene chloride, chloroform, tetrahydrofuran and the like are suitable as the solvent. It is preferred to carry out the reaction at a temperature of - 10 to 50 °C, especially - 5 °C to 5 °C.

Subsequent treatment of the product obtained above with the alkali agent can be carried out in a solvent. Suitable examples of the alkali agent include alkali metal hydroxides (e.g., sodium hydroxide, potassium hydroxide). Aqueous lower alkanol (e.g., aqueous methanol, aqueous ethanol), aqueous tetrahydrfuran, aqueous dioxane and the like are suitable as the solvent. It is preferred to carry out the reaction at a temperature of 0 to 100 °C, especially 50 to 100 °C.

Concomitantly, when the compound (I) in which ZO is a hydroxy group protected with a substituted or unsubstituted benzyloxycarbonyl group is used as the starting compound, the protecting group is removed at the same time in the above-mentioned step (i.e., treatment with the alkali agent).

According to the method of the present invention, an optically active benzylalcohol derivative (I) can be readily epimerized in a high yield. Thus, the method of the present invention is advantageous in that the undesired optical isomer of the benzylalcohol derivative (I) can be converted into the desired optical isomer thereof.

Throughout the specification and claims, the term "lower alkanoyl" should be interpreted as referring to alkanoyl having 2 to 5 carbon atoms.

The following Examples illustrate the invention.

Example 1

(1) 122.9 g of d-4-benzyloxy-α-(3,4-dimethoxyphenethylaminomethyl)benzylalcohol•(+)-N-acetyl-L-phenylalanine are addes to a mixture of 500 ml of an aqueous 2 % sodium hydroxide solution and 500 ml of methylene chloride. The mixture is stirred at 2 to 6 °C to obtain a solution containing d-4-benzyloxy-α-(3,4-dimethoxyphenethylaminomethyl)benzylalcohol as free base. To the solution are added dropwise 100 ml of an aqueous 10 % sodium hydroxide solution and a solution of 18.8 g of acetyl chloride in 100 ml of methylene chloride . The mixture is stirred at 0 to 5 °C for 40 minutes and stirred further at room temperature for 1 hour. After the reaction, the methylene chloride layer is separated therefrom, and the aqueous layer is extracted with methylene chloride.
Said methylene chloride layer and the extract is combined, washed with water, dried and evaporated to remove solvent. The residue is crystallized from isopropylether. The resultant crystals are collected by filtration and recrystallized from a mixture of isopropylether and ethyl acetate.
81.3 g of an optical isomer of 4-benzyloxy-α-(N-acetyl-3,4- dimethoxyphenethylaminomethyl)benzylalcohol are obtained as colorless plates. yield : 90 %
m.p. 97 - 98 °C
$[\alpha]_D^{20}$ - 12.77° ( C = 1, methylene chloride )

(2) 31.5 g of the product obtained above are dissolved in 120 ml of methylene chloride, and a solution of 12.5 g of thionyl chloride in 20 ml of methylene chloride is added thereto under stirring at 1 to 3 °C. The mixture is stirred at the same temperature for 15 minutes and then evaporated under reduced pressure to remove excessive thionyl chloride. The residue is dissolved in 200 ml of ethanol and the solution is added at 3 to 5 °C to a mixture of 22.4 g of sodium hydroxide, 28 ml of water and 60 ml of ethanol. The mixture is stirred at room temperature for 30 minutes and then refluxed for 3 hours. After the reaction, the reaction mixture is evaporated to remove solvent and the residue is extracted with chloroform. The extract is washed with 5 % hydrochloric acid, dried and evaporated to remove solvent. The residue is recrystallized from a mixture of ethanol and isopropylether. 25.4 g of ℓ-4-benzyloxy-α-(3,4-dimethoxyphenethylaminomethyl)benzylalcohol hydrochloride are obtained as colorless needles.
yield : 82 %
$[\alpha]_D^{20}$ - 30.1° ( C = 1, methanol )

Example 2

(1) 50 g of d-4-benzyloxy-α-(3,4-dimethoxyphenethylaminomethyl)benzylalcohol (optical purity : 58 %) are dissolved in 400 ml of ethyl acetate, and a solution of 20.3 g of potassium carbonate in 100 ml of water is added thereto at 20 to 30 °C. 15 g of acetic anhydride are added dropwise to the mixture at the same temperature under stirring and the mixture is stirred at the same temperature for 1 hour. After the reaction, the ethyl acetate layer is collected, washed with a saturated sodium chloride solution and evaporated to remove solvent. 55.2 g of an optical isomer of 4-benzyloxy-α-(N-acetyl-3,4- dimethoxyphenetylaminomethyl)benzylalcohol are obtained.

(2) 55.2 g of the product obtained above are dissolved in 250 ml of methylene chloride, and a solution of 16.9 g of thionyl chloride in 40 ml of methylene chloride is added dropwise thereto at 20 °C. The mixture is stirred at the same temperature for 15 minutes. 200 ml of water are added to the mixture at a temperature below 20 °C and said mixture is stirred for 10 minutes. After the reaction, the methylene chloride layer is collected and evaporated under reduced pressure to remove solvent. The residue is dissolved in 250 ml of methanol, and 73.5 g of an aqueous 33 % sodium hydroxide solution are added thereto at 20 °C. The mixture is stirred at room temperature for 30 minutes and refluxed for 3 hours. After cooling, the precipitated crystals are collected by filtration, washed with water and dried. 40 g of ℓ-4-benzyloxy-α-(3,4-dimethoxyphenethylaminomethyl)benzylalcohol (optical purity : 61 % ) are obtained. yield : 80 %
$[\alpha]_D^{20}$ - 14.3° ( C = 1, methanol )

Example 3

(1) 4.44 g of d-4-benzyloxy-α-(3,4-dimethoxyphenethylaminomethyl)benzylalcohol hydrochloride (optical purity : 100 %) and 2.10 g of sodium bicarbonate are dissolved in a mixture of 40 ml of water and 40 ml of methylene chloride. A solution of 1.447 g of pivaloyl chloride in 10 ml of methylene chloride is added dropwise to the solution at 5 °C under stirring and the mixture is stirred at room temperature for 30 minutes. After the reaction, the methylene chloride layer is separated, and the aqueous layer is extracted with methylene chloride. Said methylene chloride layer and the extract are combined, dried and evaporated to remove solvent. The residue is purified by silicagel column chromatography (solvent; n-hexane : ethyl acetate = 2 : 1). 4.327 g of an optical isomer of 4-benzyloxy-α-(N-pivaloyl-3,4-dimethoxyphenethylaminomethyl)benzylalcohol are obtained as pale yellow oil. yield : 88.9 %
$[\alpha]_D^{20}$ + 0.73° ( C = 5.015, methanol )

(2) 492 mg of the product obtained above are dissolved in 5 ml of methylene chloride and the solution is added dropwise at 0 to 5 °C to a solution of 360 mg of thionyl chloride in 1 ml of methylene chloride under stirring. The mixture is stirred at a room temperature for 30 minutes. After the reaction, the mixture is evaporated under reduced pressure and the residue is dissolved in 10 ml of ethanol.
A solution of 450 mg of potassium hydroxide in 1 ml of water is added to the solution and the mixture is refluxed for 1.5 hours. After the reaction, the mixture is evaporated under reduced pressure and water is added to the residue. The mixture is extracted with chloroform and the extract is washed with 10 % hydrochloric acid, dried and evaporated to remove solvent. The residue is washed with a mixture of isopropylether and ethanol ( 3 : 1 ). 440 mg of ℓ-4-benzyloxy-α-(3,4-dimethoxyphenethylaminomethyl)benzylalcohol hydrochloride (optical purity : 72.2 %) are obtained as white powder. yield : 99.1 %
m.p. 168 -170 °C
$[\alpha]_D^{20}$ - 21.58° ( C = 0.590, methanol )

**Claims**

1. A method for epimerizing an optically active benzylalcohol derivative of the formula:

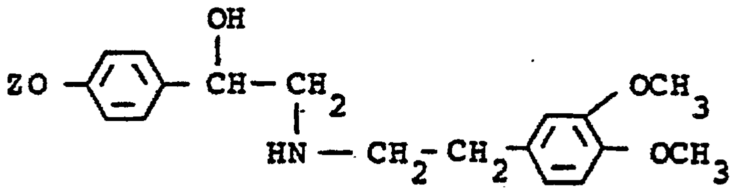

(I)

wherein ZO is a protected or unprotected hydroxy group, which comprises the steps of:
(a) condensing said optically active benzylalcohol derivative (I) or a salt thereof with a carboxylic acid compound of the formula:
$R^1COOH$ (II)
wherein $R^1CO$ is an acyl group, or a reactive derivative thereof, to give an optically active N-acylbenzylalcohol derivative of the formula:

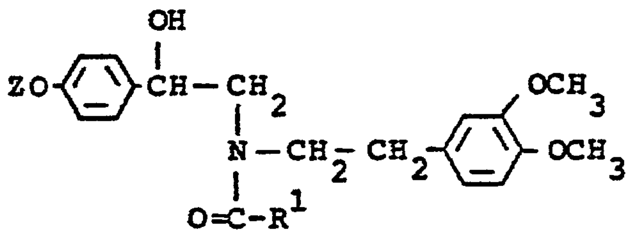

(III)

wherein $R^1CO$ and ZO are the same as defined above,
(b) subjecting the optically active N-acylbenzylalcohol derivative (III) to intramolecular cyclization in the presence of a thionyl halide, and
(c) treating the product with an alkali agent to provide the epimerized benzylalcohol derivative.

2. The method according to claim 1, wherein the protected hydroxy group is a hydroxy group which is protected with a substituted or unsubstituted benzyl group or a substituted or unsubstituted benzyloxycarbonyl group.

3. The methood according to claim 1, wherein the protected hydroxy group is benzyloxy group.

4. The method according to any one of the preceding claims, wherein $R^1CO$ is formyl group, a lower alkanoyl group, an aralkylcarbonyl or arylcarbonyl group.

5. The method according to claim 4, wherein the lower alkanoyl group is acetyl group.

6. The method according to any one of the preceding claims, wherein ZO is benzyloxy group and $R^1CO$ is a lower aklanoyl group.

7. The method according to any one of the preceding claims, wherein the benzyl alochol derivative which is epimerized is a di-isomer of compound (I).

8. The method according to claim 7, wherein the d-isomer of the compound (I) is d-4-benzyloxy-α-(3,4-dimethoxyphenethylaminomethyl)benzylalcohol.

9. The method according to claim 8 wherein the epimerization is carried out by the steps of:
(a) condensing d-4-benzyloxy-α-(3,4-dimethoxyphenethylaminomethyl)benzylalcohol or a salt thereof with acetic acid or a reactive derivative thereof,
(b) subjecting the resultant optical isomer of 4-benzyloxy-α-(N-acetyl-3,4-dimethoxyphenethylaminomethyl)benzylalcohol to intramolecular cyclization in the presence of a thionyl halide, and
(c) treating the product with an alkali metal hydroxide to give ℓ-4-benzyloxy-α-(3,4-dimethoxyphenethylaminomethyl)benzylalcohol.

10. The method according to any one of the preceding claims, wherein the condensation reaction is carried out at a temperature of - 50 to 20 °C in a solvent, the intramolecular cyclization is carried out a temperature of - 10 to 50 °C in a solvent, and the alkali metal hydroxide treatment is carried out at a temperature of 0 to 100 °C in a solvent.

## Patentansprüche

1. Verfahren zum Epimerisieren eines optisch aktiven Benzylalkoholderivates der Formel

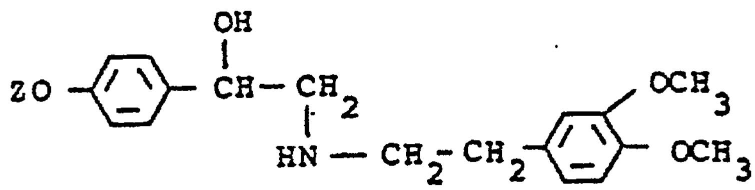

(I)

worin ZO eine geschützte oder ungeschützte Hydroxygruppe bezeichnet, umfassend die folgenden Stufen:

(a) Kondensieren des optisch aktiven Benzylalkoholderivates (I) oder eines Salzes desselben mit einer Karbonsäureverbindung der Formel

$R^1COOH$ (II)

worin $R^1CO$ eine Acylgruppe oder ein reaktives Derivat derselben bezeichnet, wobei man ein optisch aktives N-Acylbenzylalkoholderivat der Formel

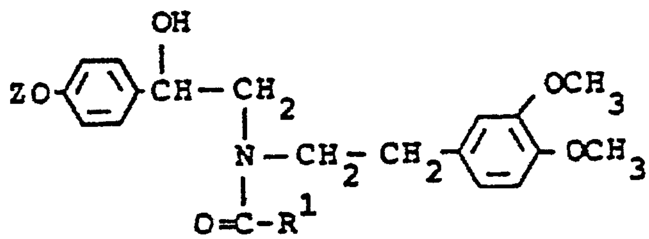

(III)

erhält, worin $R^1CO$ und ZO die vorher angegebenen Bedeutungen haben;

(b) Umsetzen des optisch aktiven N-Acylbenzylalkoholderivates (III) in einer intramolekularen Zyklisierung in Gegenwart eines Thionylhalogenids; und

(c) Behandeln des Produktes mit einem alkalischen Mittel, wobei man das epimerisierte Benzylalkoholderivat erhält.

2. Verfahren gemäss Anspruch 1, worin die geschützte Hydroxygruppe eine Hydroxygruppe ist, die durch eine substituierte oder unsubstituierte Benzylgruppe oder eine substituierte oder unsubstituierte Benzyloxycarbonylgruppe geschützt ist.

3. Verfahren gemäss Anspruch 1, worin die geschützte Hydroxygruppe eine Benzyloxygruppe ist.

4. Verfahren gemäss einem oder mehreren der vorhergehenden Ansprüche, worin $R^1CO$ eine Formylgruppe, eine Niedrigalkanoyl-, eine Aralkylcarbonyl- oder eine Arylcarbonylgruppe ist.

5. Verfahren gemäss Anspruch 4, worin die Niedrigalkanoylgruppe eine Acetylgruppe ist.

6. Verfahren gemäss einem oder mehreren der vorhergehenden Ansprüche, worin ZO eine Benzyloxygruppe und $R^1CO$ eine Niedrigalkanoylgruppe ist.

7. Verfahren gemäss einem oder mehreren der vorhergehenden Ansprüche, worin das Benzylalkoholderivat, das epimerisiert ist, ein dl-Isomer der Verbindung (I) ist.

8. Verfahren gemäss Anspruch 7, worin das d-Isomer der Verbindung (I) d-4-Benzyloxy-alpha-(3,4-dimethoxyphenethylaminomethyl) benzylalkohol ist.

9. Verfahren gemäss Anspruch 8, worin die Epimerisierung durch die folgenden Stufen ausgeführt wird:

(a) Kondensieren von d-4-Benzyloxy-alpha-(3,4-dimethoxyphenethylaminomethyl)benzylalkohol oder eines Salzes desselben mit Essigsäure oder einem reaktiven Derivat derselben;

(b) Umsetzen des entstandenen optischen Isomeren von 4-Benzyloxy-alpha-(N-acetyl-3,4-dimethoxyphenethylaminomethyl)benzylalkohol in einer intramolekularen Zyklisierung in Gegenwart eines Thionylhalogenids; und

(c) Behandlung des Produktes mit einem Alkalimetallhydroxid, wobei man l-4-Benzyloxy-alpha-(3,4-dimethoxyphenethylaminomethyl) benzylalkohol erhält.

10. Verfahren gemäss einem oder mehreren der vorhergehenden Ansprüche, worin die Kondensationsreaktion bei einer Temperatur von –50 bis 20°C in einem Lösungsmittel, die intramolekulare Zyklisierung bei einer Temperatur von –10 bis 50°C in einem Lösungsmittel und die Behandlung mit dem Alkalimetallhydroxid bei einer Temperatur von 0 bis 100°C in einem Lösungsmittel ausgeführt wird.

## Revendications

1. Méthode d'épimérisation d'un dérivé d'alcool benzylique optiquement actif de la formule:

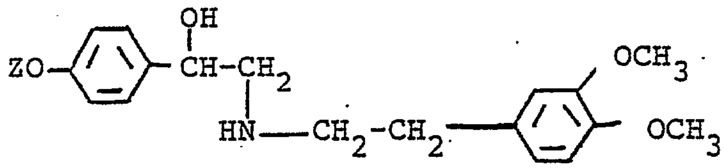

(I)

dans laquelle ZO est un groupe hydroxy protégé ou non-protégé, qui comprend les étapes consistant à:

(a) condenser le dit dérivé d'alcool benzylique optiquement actif (I) ou un sel de celui-ci avec un composé de l'acide carboxylique de la formule:

$R^1COOH$ (II)

dans laquelle $R^1$ est un groupe acyle, ou un dérivé réactif de celui-ci, pour obtenir un dérivé d'alcool N-acylbenzylique optiquement actif de la formule

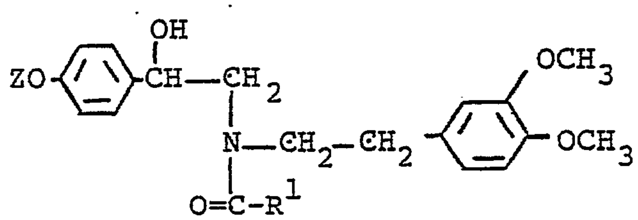

(III)

dans laquelle $R^1CO$ et ZO sont tels que définis ci-dessus

(b) soumettre le dérivé d'alcool N-acylbenzylique (III) optiquement actif à une cyclisation intramoléculaire en présence d'un halogénure de thionyle, et

(c) traiter le produit avec un agent alcalin pour fournir le dérivé d'alcool benzylique épimérisé.

2. Procédé selon la revendication 1, dans lequel le groupe hydroxy protégé est un groupe hydroxy qui est protégé par un groupe benzyle substitué ou non-substitué ou un groupe benzyloxycarbonyle substitué ou non-substitué.

3. Procédé selon la revendication 1, dans lequel le groupe hydroxy protégé est un groupe benzyloxy.

4. Procédé selon l'une quelconque des revendications précédentes, dans lequel $R^1CO$ est un groupe formyle, un groupe alcanoyle inférieur, un groupe aralkylcarbonyle ou arylcarbonyle.

5. Procédé selon la revendication 4, dans lequel le groupe alcanoyle inférieur est un groupe acétyle.

6. Procédé selon l'une quelconque des revendications précédentes, dans lequel ZO est un groupe benzyloxy et $R^1CO$ est un groupe alcanoyle inférieur.

7. Procédé selon l'une quelconque des revendications précédentes, dans lequel le dérivé d'alcool benzylique qui est épimérisé est un d-isomère du composé (I).

8. Procédé selon la revendication 7, dans lequel le d-isomère du composé (I) est l'alcool d-4-benzyloxy-α-(3,4-diméthoxyphénéthylaminométhyl)benzylique.

9. Procédé selon la revendication 8, dans lequel on effectue l'épimérisation en procédant aux étapes consistant à:

(a) condenser l'alcool d-4-benzyloxy-α-(3,4-diméthoxyphénéthylaminométhyl)benzylique ou un sel de celui-ci avec de l'acide acétique ou un dérivé réactif de celui-ci,

(b) soumettre l'isomère optique de l'alcool 4-benzyloxy-α-(N-acétyl-3,4-diméthoxyphénéthylaminométhyl)benzylique en résultant à une cyclisation intramoléculaire en présence d'un halogénure de thionyle et

(c) traiter le produit avec un hydroxyde de métal alcalin pour obtenir l'alcool l-4-benzyloxy-a-(3,4-diméthoxyphénéthylaminométhyl)benzylique.

10. Procédé selon l'une quelconque des revendications précédentes dans lequel la réaction de condensation est effectuée dans un solvant, à une température de –50 à 20°C, la cyclisation intramoléculaire est effectuée dans un solvant, à une température de –10 à 50°C et le traitement à l'hydroxyde de métal alcalin est effectué dans un solvant, à une température de 0 à 100°C.